# EUROPEAN PATENT APPLICATION

(11) **EP 4 759 932 A1**
(43) Date of publication of application: **17.06.2026**
(21) Application number: 24937641.9
(22) Date of filing: 30.09.2024
(51) Int. Cl.: C12N 15/88, C12N 5/10, C12N 15/12, C12N 5/074

(54) **MRNA-LNP-BASED REPROGRAMMING METHOD FOR INDUCED PLURIPOTENT STEM CELLS**

(30) Priority: 30.04.2024 CN 202410540412
(71) Applicant: UBRIGENE (MA) BIOSCIENCES INC., Dover, DE 19901 (US)
(72) Inventor: ZHAO, Sheng, Suzhou, Jiangsu 215000 (CN); TANG, Chuanqing, Suzhou, Jiangsu 215000 (CN); SUN, Xiulian, Suzhou, Jiangsu 215000 (CN)
(74) Representative: Huang, Liwei
(86) International application number: PCT/CN2024/122751
(87) International publication number: WO 2025/227611

(57) **Abstract**

The present invention provides a reprogramming method for an induced pluripotent stem cell based on mRNA-LNP. Specifically, a nanoliposome, i.e., mRNA cocktail-LNP, is prepared by encapsulating an mRNA cocktail of a plurality of reprogramming factors with a cationic lipid. A certain amount of the nanoliposome containing the reprogramming factors is added dropwise to a somatic cell in a feeder-free system to obtain a reprogrammed induced pluripotent stem cell, achieving a safer iPSCs reprogramming technology with low cost, low toxicity, high efficiency, stability, and reproducibility.

## Description

### TECHNICAL FIELD

The present invention relates to regenerative medicine and cells, and in particular to a reprogramming method for a human somatic cell into a human induced pluripotent stem cell (iPSC) using mRNA-LNP.

### BACKGROUND ART

The scientific researchers have been focused on fields of regenerative medicine and cell therapy because of its great potential to solve many diseases and health problems. In recent years, the research of stem cells has intensively developed. Stem cells can self-renew and differentiate into various cell types, thereby being utilized as an important resource in the field of regenerative medicine. The method for reprogramming a human somatic cell into an induced pluripotent stem cell (iPSC) is an important technical means to obtain a stem cell. In 2006, Yamanaka's team (Takahashi K, Yamanaka S. Induction of pluripotent stem cells from mouse embryonic and adult fibroblast cultures by defined factors. Cell 126: 663 - 676, 2006.) invented a cocktail of four genes, Oct4, Sox2, Klf4, and c-Myc, and successfully reprogrammed terminally differentiated skin fibroblasts into induced pluripotent stem cells (iPSCs) through viral infection. A year later, James Thomson (Yu J, Vodyanik MA, Smuga-Otto K, Antosiewicz-Bourget J, Frane JL, Tian S, Nie J, Jonsdottir GA, Ruotti V, Stewart R, Slukvin II, Thomson JA. Induced pluripotent stem cell lines derived from human somatic cells. Science 318: 1917-1920, 2007.) successfully reprogrammed human fibroblasts into iPSCs by episomal plasmid transfection using a combination of four different factors (Oct4, Sox2, Nanog and Lin28). While these above conventional technologies can overcome ethical issues while expanding therapeutic potential, the clinical application of iPSCs faces many challenges, among which the integration of exogenous genes into the genome brings great obstacles to the therapeutic application of induced pluripotent stem cells. Therefore, it is important to develop a simple and reproducible method to generate integration-free clinical-grade iPSCs.

Existing non-integration reprogramming technologies mainly include the use of excisable lentiviral and transposon vectors, episomal and adenoviral vectors to obtain iPSCs. Alternatively, iPSCs can be obtained using DNA-free methods, such as sequential protein transduction using recombinant proteins containing cell-penetrating peptide fragments or transgene delivery using Sendai virus. However, these above methods inevitably introduce at least some drawbacks, such as generating non-human special functional nucleic acids to a greater or lesser extent, thereby provoking still potential risks and uncertainties. The non-integrating iPSCs strategies designed so far still have limitations. For example, the DNA transfection method carries the risk of gene recombination or insertional mutations, the production and removal of recombinant proteins are challenging, the use of Sendai virus requires strict control, and the current induction efficiency is low.

It has been demonstrated that non-integrated iPSCs can also be obtained by repeated transfection of mRNA. However, the existing mRNA transfection technology is costly and relies on expensive transfection reagents such as Lipofectamine RNAiMAX. In addition, the transfection reagent is highly toxic, and the efficiency of transfection of mRNA is low. It is usually necessary to develop a variety of microRNAs to improve the reprogramming efficiency, thereby increasing the cost and potential other risks and uncertainties. Therefore, it is crucial to develop a simple and effective reprogramming method.

There is no systematic research report in the field of cell reprogramming, and it is still challenging to establish efficient and reproducible reprogramming technology by using nanoliposomes. Thus, it is necessary to develop a mRNA nanoliposome (mRNA-LNP) delivery system having a wide range of application prospects in the biomedical field, especially in the fields of gene therapy and vaccine development.

### SUMMARY OF THE INVENTION

The inventors of the present invention have discovered that using a nanoliposome (LNP) delivery system to deliver an mRNA cocktail containing a plurality of reprogramming genes (mRNA-LNP) not only avoids the risks of gene integration and gene contamination, but also has simpler components and does not require the addition of microRNA or the use of expensive transfection reagents to achieve reprogramming. The mRNA cocktail-LNP prepared by the present invention not only successfully reprograms somatic cells (such as human dermal fibroblasts) to produce iPSCs, but also has extremely high reprogramming efficiency.

The present invention provides a reprogramming method for an induced pluripotent stem cell. An exemplary method comprises:
S1: Preparing a nanoliposome by using a cationic lipid to encapsulate a cocktail of mRNAs of reprogramming factors;
The method for preparing the nanoliposome mainly involves mixing materials for encapsulating nucleic acids, such as cationic lipids, with a buffer containing nucleic acids. Currently, mixing methods suitable for small-volume LNP production in the laboratory include but are not limited to microfluidic mixing devices or chips, T-type or y-type mixers, ethanol injection, and manual mixing. In some embodiments, the preparation method utilizes a microfluidic chip.

The nanoliposome is mainly composed of the following parts in structure:
(1) Cationic lipids: Cationic lipids are the most critical components in LNP delivery systems. Cationic lipid molecules typically contain one or more positively charged head groups, one hydrophobic tail (usually a long chain hydrocarbon group or a saturated/unsaturated fatty acid chain), and a linking portion (such as a glycerol backbone) that connects the head group to the tail's hydrophobic chain.
(2) Helper lipids: Helper lipids are typically uncharged lipids, such as phosphatidylcholine. They are also composed of a head group, a hydrophobic tail, and a structure connecting the two. Helper lipids help stabilize the structure of liposomes, reduce the positive charge density on the surface of cationic liposomes, alleviate possible toxicity, and can adjust the fluidity and size of liposomes to make them more suitable for drug delivery.
(3) Cholesterol (if any): Cholesterol is a sterol lipid containing one polycyclic structure and one hydrophobic tail. Cholesterol is embedded in the bilayer of liposomes, which can enhance the stability of liposomes, reduce the leakage of drugs, adjust the fluidity of liposomes, and may help the fusion process of liposomes with cell membranes.
(4) Targeting ligands (if any): Targeting ligands can be antibodies, proteins, polypeptides, or small molecules that can specifically bind to receptors or other molecules on the cell surface. Targeting ligands help direct liposomes to specific types of cells, improving drug selectivity and intracellular delivery efficiency.
(5) Polyethylene glycol (PEG) modification (if any): PEG is a widely used polymer, and is a chain structure composed of repeating ethylene glycol units.

In some embodiments, the cationic lipid is one or more selected from SM102 (CAS: 2089251-47-6), MC3 (CAS: 1224606-06-7), ALC0315 (CAS: 2036272-55-4), and ALC0159 (CAS: 1849616-42-7). In some embodiments, the cationic lipid is selected from SM102.

The LNPs may comprise one or more cationic lipids, non-cationic lipids, and/or PEG-modified lipids. In some embodiments, the LNPs may comprise at least one of the following cationic lipids: SM102, MC3, ALC0315, or ALC0159. In some embodiments, the LNPs further comprise cholesterol and/or PEG-modified lipids.

The conventional four reprogramming factors are Oct4, Sox2, Klf4 and c-Myc, i.e. OSKM. The method provided by the present invention comprises a mRNA cocktail of five or more reprogramming factors. Specifically, the mRNA cocktail of reprogramming factors is a cocktail of 5 or more factors selected from mRNA-hOCT3/4, mRNA-hSox2, mRNA-hKlf4, mRNA-hcMyc, mRNA-hLin28, mRNA-hNanog, and mRNA-hGlis.

In some embodiments, the mRNA cocktail of reprogramming factors is a mRNA cocktail of five reprogramming factors; specifically, it can be a cocktail of the four factors mRNA-hOCT3/4, mRNA-hSox2, mRNA-hKlf4, and mRNA-hcMyc, with one of the three factors mRNA-hLin28, mRNA-hNanog, and mRNA-hGlis. In some embodiments, the mRNA cocktail of reprogramming factors is a cocktail of mRNA-hOCT3/4, mRNA-hSox2, mRNA-hKlf4, mRNA-hcMyc and mRNA-hGlis, i.e., five factors OSKMG. In some embodiments, the mRNA cocktail of reprogramming factors is a cocktail of mRNA-hOCT3/4, mRNA-hSox2, mRNA-hKlf4, mRNA-hcMyc, and mRNA-hLin28, i.e. five factors OSKML.

S2: Inducing reprogramming of a somatic cell using the nanoliposome to obtain a reprogrammed induced pluripotent stem cell.

A certain amount of the nanoliposome containing the reprogramming factors is added dropwise to a somatic cell in a feeder-free system to obtain a reprogrammed human induced pluripotent stem cell.

The somatic cell refers to a cell that has differentiated and has specific functions, as opposed to a stem cell. The somatic cell makes up the various tissues and organs of the human body and plays an important role in maintaining life and function. The somatic cell is usually no longer able to differentiate into other types of cells.

In some embodiments, the somatic cell is one or more selected from a skin fibroblast, an oral epithelial cell, a hepatocyte, a gastric cell, a keratinocyte, an adipocyte, a muscle cell or a blood cell.

In some embodiments, the somatic cell is derived from a mammal. Specifically, the mammal may be a human, or another mammal such as a monkey, a cow, a horse, a sheep, or a murine. Specifically, the somatic cell is a human dermal fibroblast (HDF).

The culture system of iPSCs can be divided into two types: feeder layer system and feeder-free system according to the presence or absence of feeder cells. Feeder cells are composed of a layer of cells that cannot divide, so they are sometimes also called feeder layer cells. They provide extracellular secretory substances that help another type of cell to proliferate. The feeder layer culture system is a traditional stem cell culture system. However, feeder layer cells have the risk of heterologous contamination while maintaining the stem cell state. The feeder-free culture system is a culture system that has gradually emerged in recent years for the clinical application of stem cells. It can avoid contamination by heterologous cells. In some embodiments, the feeder-free system is selected from a basement membrane matrix with an animal-derived component, a vitronectin adhesion matrix without an animal-derived component, and a recombinant human laminin matrix without an animal-derived component.

The present invention further provides a reprogrammed human induced pluripotent stem cell prepared by any of the above-mentioned reprogramming methods. In some embodiments, the human somatic cells used for reprogramming are human dermal fibroblasts. Human dermal fibroblasts are a common type of somatic cells, which mainly exist in the dermis of human body and are one of the main cell types in dermal tissues. The dermis is the deep tissue of the skin that contains elastic fibers and collagen fibers, which play a role in support, protection, and nutrition.

The present invention provides a reprogramming method for a human induced pluripotent stem cell, which comprises that a nanoliposome mRNA cocktail-LNP is prepared by using a cationic lipid to encapsulate a mRNA cocktail of reprogramming factors; and a certain amount of the nanoliposome containing the reprogramming factors is added dropwise to a human somatic cell in a feeder-free system to obtain a reprogrammed human induced pluripotent stem cell, achieving a safer iPSCs reprogramming technology with low cost, low toxic. This technology successfully reprogrammed human somatic cells to produce high-quality and high-safety iPSCs, providing new strategies and tools for research in the field of cell reprogramming. In the future, this technology is expected to be widely used in the field of biomedicine, bringing revolutionary changes to the fields such as disease treatment, tissue regeneration, and personalized medicine.

### BRIEF DESCRIPTION OF THE DRAWINGS

In order to better understand the present invention and to more clearly show how to implement the present invention, the features of embodiments according to the present invention will now be described by way of example with reference to the accompanying drawings for illustration only and thus are not intending to limit the present invention, wherein:

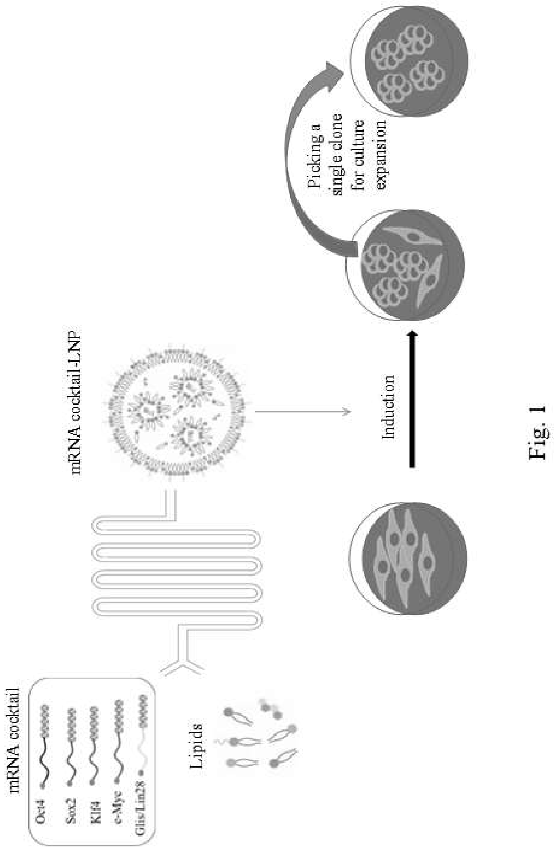

FIG. 1: Process flow chart of reprogramming technology based on mRNA-LNP according to one embodiment of the present invention, wherein: A is a schematic diagram of preparing mRNA cocktail-LNP based on microfluidic chip; B is a schematic diagram of the morphological changes of human dermal fibroblasts reprogrammed into iPSCs.

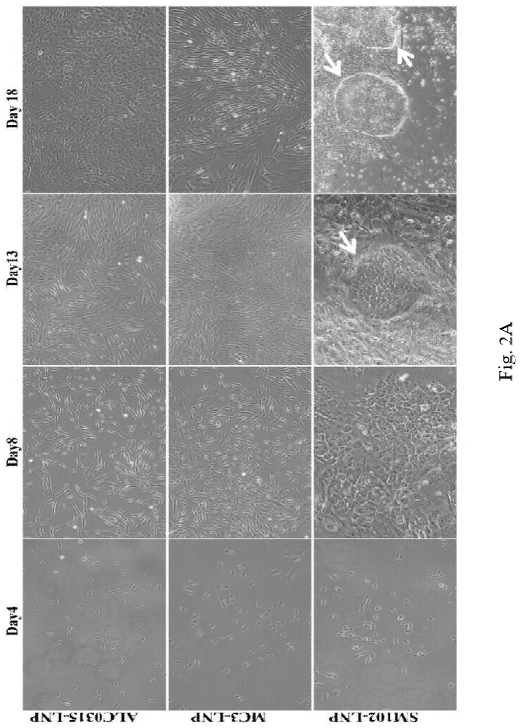

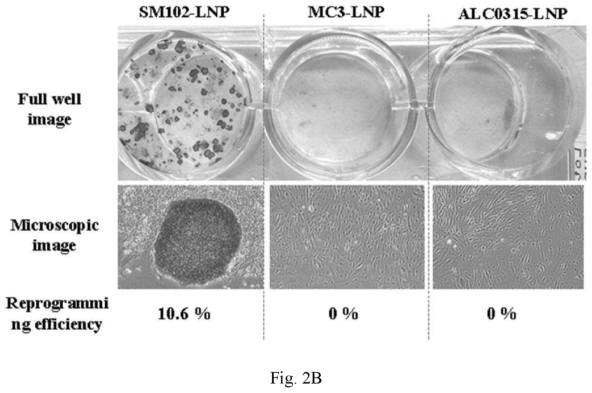

FIGs. 2A-2B: Characterization of programming induced by RNA cocktail-LNPs with different cationic lipid formulations according to the above embodiment of the present invention, wherein: FIG. 2A is a diagram of cell morphology changes during reprogramming induced by RNA cocktail-LNPs containing three different cationic lipids, with arrows indicating typical iPSC colonies; FIG. 2B is the AP staining identification diagram, in which AP positive is iPSCs clone, and the reprogramming efficiency calculation formula is: Reprogramming efficiency = number of AP positive clones/number of initial seeded cells × 100%.

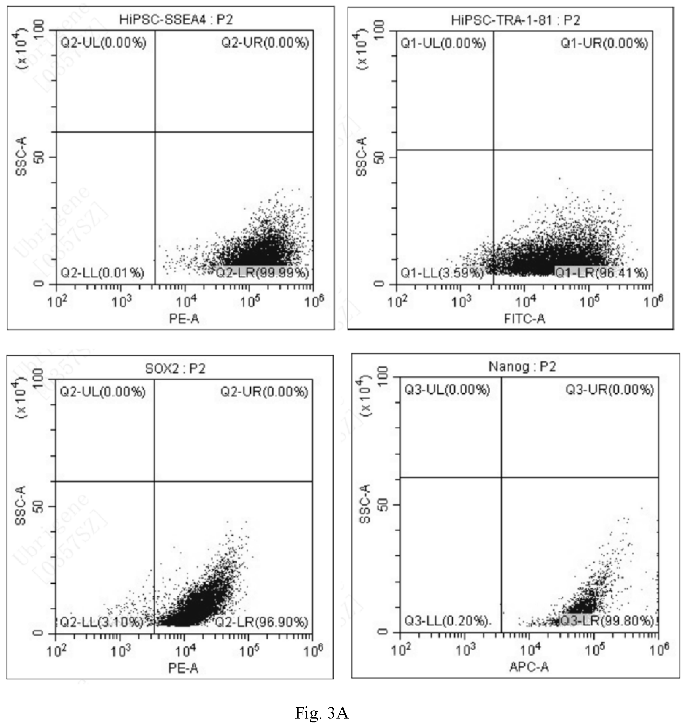

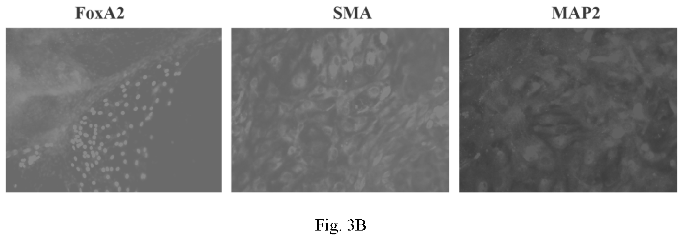

FIGs. 3A-3B: Identification and characterization of stemness of iPSCs obtained by reprogramming according to the above embodiment of the present invention, wherein: FIG. 3A is the detection of pluripotent gene expression in iPSCs by flow cytometry; FIG. 3B is the identification of the self-differentiation ability of iPSCs by immunofluorescence staining.

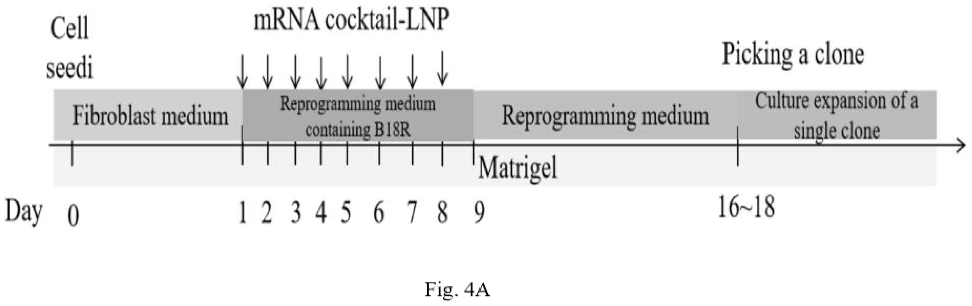

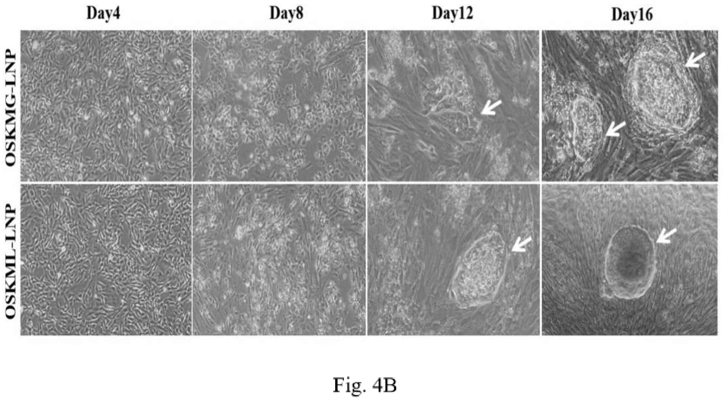

FIGs. 4A-4B: Characterization of programming induced by RNA cocktail-LNPs with different reprogramming factor formulations according to the above embodiment of the present invention, wherein: FIG. 4A is the flow chart of LNP-induced reprogramming; FIG. 4B is a diagram of cell morphology changes during reprogramming induced by two mRNA cocktail-LNPs containing five factors, with arrows indicating typical iPSC colonies.

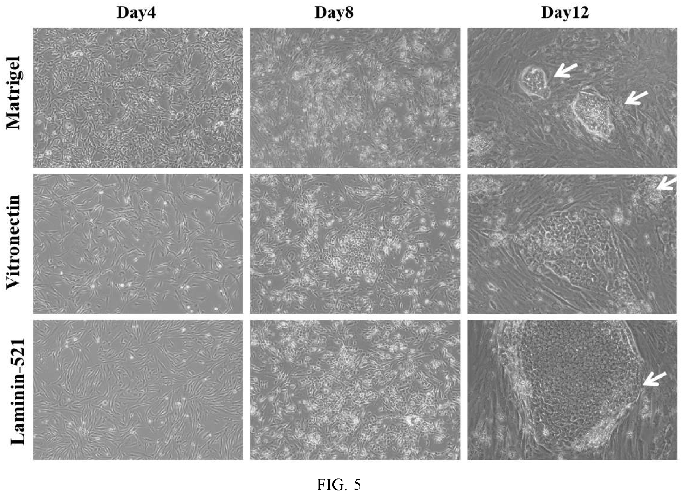

FIG. 5: Images of morphological changes during reprogramming cells into iPSCs based on mRNA-LNP using different matrigels according to the above embodiment of the present invention.

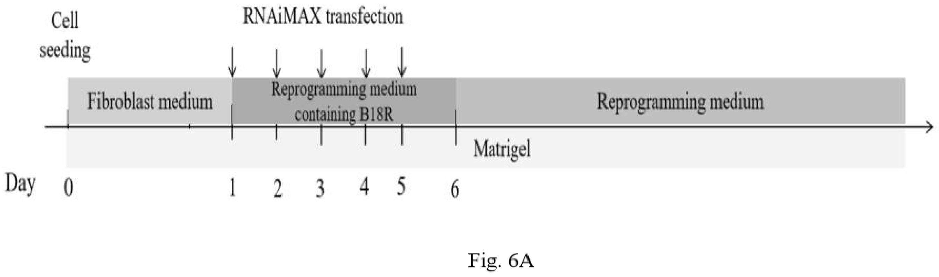

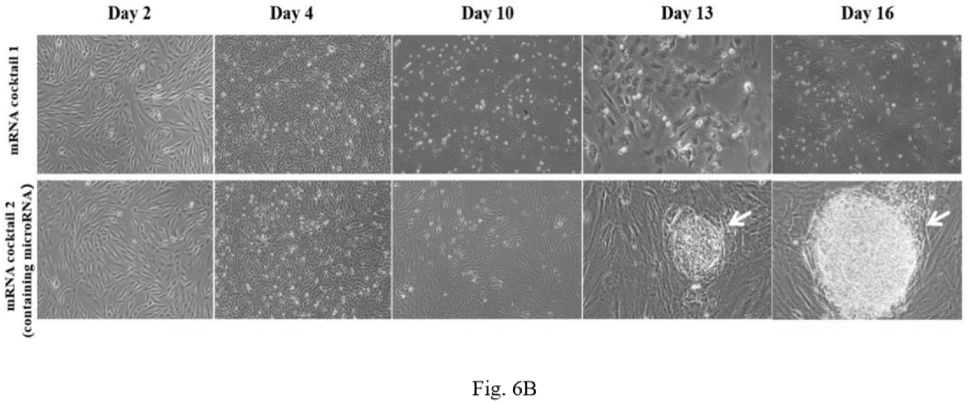

FIGs. 6A-6B: Characterization of iPSC reprogramming by transfection mode according to the above embodiment of the present invention, wherein: FIG. 6A is the reprogramming flow chart of the transfection mode; FIG. 6B is a diagram of cell morphology changes during reprogramming induced by different RNA cocktails, with arrows indicating typical iPSC colonies.

### DETAILED DESCRIPTION OF EMBODIMENTS

Definitions: In order to provide a clear and consistent understanding of the terms used in the specification of the present invention, some definitions are provided below. Furthermore, unless otherwise specified, all technical and scientific terms used in the present invention have the same meaning as commonly understood by one of ordinary skill in the art to which the present invention belongs.

When used in conjunction with the term "comprise" in the claims and/or specification, the use of the term "a" may mean "one," but it is also known with the meanings of "one or more," "at least one," and "one or more than one." Similarly, the term "another" may mean at least a second or a plurality of.

As used herein, the terms "comprise" (and any forms of "comprise", such as "comprising" and "comprised"), "having" (and any forms of "having", such as "include" and "contain") are inclusive and open-ended, and do not exclude additional unlisted elements or processing steps.

Examples of the present invention will be more readily understood by reference to the following examples, which are used to illustrate the present invention and should not be construed as limiting the scope of the present invention in any way.

Unless otherwise defined or the context clearly dictates otherwise, all technical and scientific terms used herein have the same meaning as commonly understood by one of ordinary skill in the art to which the present invention belongs. It should be understood that any methods and materials similar or equivalent to those described herein can be used in the practice or testing of the present invention.

The present invention provides a method for inducing human dermal fibroblasts into iPSCs based on mRNA-LNP. The present invention is further illustrated in detail by exemplifying an mRNA cocktail comprising at least 5 reprogramming factors. The illustrated embodiments are not intended to limit the scope of the claims of the present invention but are merely intended to illustrate certain embodiments. Any variations in the exemplary methods that occur to those skilled in the art are intended to fall within the scope of the present invention. Other embodiments that can be made in accordance with the principles of the present invention all fall within the scope defined by the claims of the present invention.

Experimental methods not specifically described in the present invention are performed according to the specific methods in the book Molecular Cloning: A Laboratory Manual (Fourth Edition) by Joseph Sambrook or the instructions of relevant products. When used herein, all terms in the present invention are to be understood in their ordinary meanings known in the art, unless otherwise indicated. The biological agents used in the present invention are commercially available unless otherwise specified.

### Example 1: Preparation of mRNA cocktail-LNP with different lipid formulations

### 1) In vitro transcription to obtain mRNA

Plasmid vectors containing reprogramming factor sequences were obtained by gene synthesis (General Biol (Anhui) Co., Ltd.). The reprogramming factors were hOct3/4 (SEQ ID NO: 1/7), hKlf4 (SEQ ID NO: 2/8), hSox2 (SEQ ID NO: 3/9), hcMyc (SEQ ID NO: 4/10), and hLin28 (SEQ ID NO: 5/11). The plasmid is then digested using restriction endonuclease to obtain a linearized plasmid DNA template, and NsiI restriction endonuclease is selectively used in this example. mRNA was synthesized by in vitro transcription from the linearized plasmid DNA template and capping using a T7 high yield RNA synthesis kit (Hzymes Biotechnology Co., Ltd.). After in vitro transcription, the reaction product was treated with DNase I (Hzymes Biotechnology Co., Ltd.) for 30 min to remove the DNA template. After DNase I treatment, the mRNA is purified. The purification method can be column purification, ethanol precipitation, lithium chloride precipitation, HPLC purification, etc. In this example, lithium chloride precipitation is selectively used.

### 2) Preparation of mRNA-LNPs with different cationic lipids based on microfluidic chips

Three groups of formulations A, B, and C were set up, among which the formulations of five-factor mRNA cocktail all consisted of mRNA-hOCT3/4, mRNA-hSox2, mRNA-hKlf4, mRNA-hcMyc and mRNA-hLin28, i.e. OSKML. The formulation A used SM102 as cationic lipid to prepare mRNA cocktail-SM102-LNP. The formulation B used MC3 as cationic lipid to prepare mRNA cocktail-MC3-LNP. The formulation C used ALC0315 as cationic lipid to prepare mRNA cocktail-ALC0315-LNP.

The schematic diagram of the process for generating iPSCs based on mRNA-LNP reprogramming is shown in FIG. 1. Raw materials such as lipids, single nucleic acids or multiple nucleic acids were injected into a microfluidic mixing chip through a microfluidic device, respectively. Then, the raw materials were fully mixed and emulsified in the chip by adjusting parameters such as pressure, flow rate, and temperature, so as to form a stable LNP. The prepared LNPs were collected through the outlet of the microfluidic device and then concentrated by ultrafiltration to obtain the final mRNA cocktail-SM102-LNP corresponding to the formulation A, mRNA cocktail-MC3-LNP corresponding to the formulation B, and mRNA cocktail-ALC0315-LNP corresponding to the formulation C.

### Example 2: Induction of iPSC reprogramming by mRNA cocktail-LNPs with different lipid formulations

LNPs obtained from three different lipid formulations, A, B, and C in Example 1 were delivered into HDF cells for reprogramming to generate iPSCs. Materials included: primary culture of HDF cells; prepared LN-521 coated plates (BioLamina); preprogramming induction medium (StemCell); sterile pipette tips (1000 µL, 200 µL, and 10 µL). The specific implementation is as follows:

### 1) Day 0: HDF cell seeding

HDF cells were seeded in an LN-521-coated 12-well plate at a density of 1000-5000 cells/well. The cells were cultured overnight in DMEM high glucose medium containing 10% fetal bovine serum and 1% Glutamax.

### 2) Day 1-8: Dropwise addition of LNPs

The medium was replaced with reprogramming induction medium containing 200 ng/mL B 18R recombinant protein. LNPs were added dropwise to the cells, and the amount of LNPs in this example was selectively 0.5 to 2 µg total mRNA/well, the dropwise addition operation was repeated for 6 to 8 days.

Cell morphology changes were continuously observed. The results are shown in FIG. 2A.

The results showed that there was no obvious change in cell morphology during the reprogramming process of LNP containing MC3 and ALC0315, and no colonies with iPSC-like morphology were found in culture up to day 18 of reprogramming. Cells treated with SM102-LNP showed obvious cell morphology changes in the early stages of reprogramming, with no obvious cell death during the culture process. In addition, colonies with typical iPSC morphology could be observed on the day 13 of reprogramming. The iPSC clone formation rate was high, indicating that the mRNA cocktail-SM102-LNP containing the reprogramming factors provided by the present invention could efficiently induce iPSCs.

### 3) Alkaline phosphatase (AP) staining

In order to characterize iPSCs and calculate the reprogramming efficiency, the present invention used an alkaline phosphatase staining kit (Yeasen Biotechnology (Shanghai) Co., Ltd.) to perform AP staining on samples on day 18 of reprogramming, and the specific operation was performed according to the manufacturer's instructions.

AP activity is mainly expressed in embryonic stem cells and some somatic stem cells, but not in normal human cells. After AP transfection, cells that formed iPSCs clones had AP activity, while cells that did not form iPSCs clones were negative for AP activity after staining. The staining results are shown in FIG. 2B.

The results showed that no AP-positive cells were detected in mRNA cocktail-MC3-LNP group and mRNA cocktail-ALC0315-LNP group, while there were a substantial number of AP-positive cells in mRNA cocktail-SM102-LNP group, and the reprogramming efficiency reached 10.6% (number of AP-positive clones/density of initial seeded cells × 100%). This result further proved that the mRNA cocktail-SM102-LNP containing reprogramming factors provided by the present invention could induce iPSCs, and the reprogramming efficiency was relatively high, reaching 10%. In addition, the LNP provided by the present invention had no obvious toxicity and was relatively simple in composition.

### Example 3: Identification of stemness of iPSCs obtained by reprogramming

When the iPSCs induced by mRNA cocktail-SM102-LNP grow to a large enough size, the clones were picked out for culture expansion, and the iPSCs were identified. The identification methods included but were not limited to stem cell pluripotency marker detection, AP staining, in vitro differentiation experiments, etc. This example selectively tested the stemness and differentiation ability of iPSCs.

### 1) Detection of pluripotent gene expression by flow cytometry

The stemness detection methods of iPSCs include RT-qPCR, immunofluorescence staining, and flow cytometry to detect the expression of pluripotent genes in pluripotent cells. In this example, flow cytometry was selectively used to detect pluripotent genes, and stemness markers such as SSEA4, TRA-1-18, Sox2, and Nanog were selectively used.

The iPSCs were dissociated into single cell suspensions using EDTA (Thermofisher). The iPSCs were stained using flow cytometry antibodies such as SSEA4, TRA-1-18, Sox2, and Nanog, and HDF cells were stained as a negative control. The stained cells were then analyzed using a BD flow cytometer, and the flow cytometry results are shown in FIG. 3A and Table 1.

The results showed that all stemness markers in the iPSCs obtained by reprogramming were positive. Compared with the control group, the SSEA4-, TRA-1-18-, Sox2- and Nanog-positive cells in iPSCs were 99.99%, 96.41%, 96.9%, and 99.8%, respectively. This result indicated that the expression of pluripotent genes in iPSCs obtained by reprogramming was normal and the stemness was good.

**Table 1 Results of detection of different markers by flow cytometry**

| **Antibody Name** | **SSEA4** | **TRA-1-81** | **SOX2** | **Nanog** |
|---|---|---|---|---|
| Positivity rate | 99.99% | 96.41% | 96.90% | 99.80% |

### 2) Detection of the self-differentiation performance of iPSCs in vitro by immunofluorescence staining

Generally, stem cells enable self-differentiation, to form embryoid bodies (EB) and self-differentiate into three germ layers. The iPSCs obtained by reprogramming were digested into small clumps using EDTA and seeded into differentiation medium to allow them to self-aggregate to form EB spheres. The selective differentiation medium for this example was DMEM/F12 medium containing 20% KOSR, 1% NEAA, 1% Glutamax-1 and 100 µM β-mercaptoethanol. After EB spheres were formed and grown for 7 days, they were seeded into a 12-well plate coated with 0.1% Gelatin and cultured for another 7 days in differentiation medium containing 5% fetal bovine serum. Then, the cells were fixed with 4% paraformaldehyde and stained with antibodies against differentiation markers of three germ layers. The selective antibodies in this example were endoderm: FoxA2; mesoderm: SMA; ectoderm: MAP2. After secondary antibody staining and washing, the cells were observed and photographed using a fluorescence microscope. The staining results are shown in FIG. 3B. The results showed that the iPSCs obtained by reprogramming could form EB spheres in vitro and successfully differentiate into cells of three germ layers, indicating that the cells obtained by reprogramming met the identification criteria of iPSCs and had self-differentiation ability.

### Example 4: iPSC reprogramming by mRNA cocktail-LNPs with different reprogramming factor formulations

This example used LNPs to deliver different reprogramming mRNA cocktail formulations into HDF cells for reprogramming to generate iPSCs. The selected two mRNA cocktail formulations containing five factors were mRNA-hOCT3/4, mRNA-hSox2, mRNA-hKlf4, mRNA-hcMyc, and mRNA-hGlis (SEQ ID NOs: 6/12) (OSKMG) and mRNA-hOCT3/4, mRNA-hSox2, mRNA-hKlf4, mRNA-hcMyc, and mRNA-hLin28 (OSKML). The selective cationic lipid formulation for preparing LNPs in this example was SM102. The selective reprogramming flowchart in this example is shown in FIG. 4A, and the specific implementation is as follows:

### 1) Day 0: HDF cell seeding

HDF cells were seeded in a matrigel-coated 6-well plate at a density of 1000-5000 cells/well. The cells were cultured overnight in DMEM high glucose medium containing 10% fetal bovine serum and 1% Glutamax.

### 2) Day 1-8: Dropwise addition of LNPs

The medium was replaced with reprogramming induction medium containing 200 ng/mL B18R recombinant protein. LNPs were added dropwise to the cells, and the amount of LNPs in this example was selective 0.5 to 2 µg total mRNA/well, the dropwise addition operation was repeated for 6 days.

The cell morphology changes were continuously observed, and the cell morphology changes were observed. The results are shown in FIG. 4B. The results showed that obvious iPSCs clones appeared on the day 16 of reprogramming, indicating that both OSKMG and OSKML formulations of mRNA-LNP could reprogram HDF cells into iPSCs.

### Example 5: Reprogramming cells into iPSCs based on mRNA-LNP using different matrigels

This example presents a method for reprogramming cells into iPSCs based on mRNA-LNP using different matrigels. The matrigel used in this example was selectively selected from conventional Matrigel with an animal-derived component, a vitronectin adhesion matrix without an animal-derived component (Vitronectin), and a recombinant human laminin matrix without an animal-derived component (Laminin-521). The mRNA-LNPs used in this example were mRNA cocktail-LNP prepared using SM102. The specific implementation is as follows:

### 1) Day 0: HDF cell seeding

HDF cells were seeded in a matrigel-coated 6-well plate at a density of 1000-5000 cells/well. The cells were cultured overnight in DMEM high glucose medium containing 10% fetal bovine serum and 1% Glutamax.

### 2) Day 1-8: Dropwise addition of LNPs

The medium was replaced with reprogramming induction medium containing 200 ng/mL B18R recombinant protein. LNPs were added dropwise to the cells, and the amount of LNPs in this example was selectively 0.5 to 2 µg total mRNA/well, the dropwise addition operation was repeated for 6 to 8 days.

The cell morphology changes were continuously observed, and the results are shown in FIG. 5. The results showed that three different matrigels could successfully reprogram human dermal fibroblasts (HDF) based on mRNA-LNP, and the cells had obvious morphology changes and formed typical iPSC clones. The arrows of FIG. 5 indicated typical iPSC colonies.

### Example 6: Reprogramming cells into iPSCs by transfection

For comparison, commonly used transfection reagents were tested for mRNA reprogramming. Transfection reagents could be selected, such as liposome transfection, calcium transfection, or PEI transfection. In this example, Lipofectamine RNAiMAX transfection reagent (Invitrogen) was selectively used.

This example describes a method for generating iPSCs by transfecting human dermal fibroblasts (HDFs) with mRNA for reprogramming. Materials used included: HDF cells (donated by other laboratories); Lipofectamine RNAiMAX transfection reagent (Invitrogen); Opti-MEM (Gibco); prepared LN-521 coated plates; reprogramming induction medium; sterile 1.5 mL Eppendorf tubes; sterile pipette tips (1000 µL, 200 µL, and 10 µL). The reprogramming mRNAs and microRNAs included: (1) mRNA-hOCT3/4; (2) mRNA-hKlf4; (3) mRNA-hSox2; (4) mRNA-hcMyc; (5) mRNA-hLin28; (6) mRNA-hNanog (SEQ ID NO: 13/14); (7) mRNA-hGlis; and (8) microRNA302/367.

The schematic diagram of reprogramming by transfection is shown in FIG. 6A. The specific implementation is as follows:

### 1) Day 0: HDF cell seeding

HDF cells were seeded in an LN-521-coated 6-well plate at a density of 50,000 cells/well.

### 2) Day 1-5: Lipofectamine RNAiMAX transfection

The medium was replaced with reprogramming induction medium containing 200 ng/mL B18R recombinant protein. The mRNA cocktail was prepared after changing the medium. In this example, 2 mRNA cocktails were prepared. mRNA cocktail 1 did not contain microRNA and mainly included (1) mRNA-hOCT3/4; (2) mRNA-hKlf4; (3) mRNA-hSox2; (4) mRNA-hcMyc; (5) mRNA-hLin28; (6) mRNA-hNanog; and (7) mRNA-hGlis. mRNA cocktail 2 was based on mRNA cocktail 1 with the addition of microRNA. In this example, microRNA 302/367 was selectively used. Subsequently, transfection was performed using RNAiMax liposome reagent according to the manufacturer's instructions.

4 additional transfections were performed on days 2 to 5 of reprogramming (see schematic FIG. 6A).

### 3) Day 6-18: The medium was changed to reprogramming induction medium.

Cell morphology changes were continuously observed. The results are shown in FIG. 6B. The results showed that mRNA cocktail 1 without microRNA caused massive cell death in the middle and late stages of reprogramming, and no colonies with iPSCs-like morphology were found. The mRNA cocktail 2 containing microRNA could induce iPSCs, but there was also a lot of cell death, and the number of iPSC clones ultimately formed was small, with a calculated reprogramming efficiency of 0.033% (number of iPSC colonies/density of initial seeded cells × 100%). This result further proved that the current commonly used transfection reagents in the method had many limitations, including high cytotoxicity of transfection reagents, low reprogramming efficiency, complex components, and the need for the presence of microRNA.

As discussed above, the present invention provides a reprogramming method for human induced pluripotent stem cells. This method not only utilizes LNP technology to deliver mRNA, but also has good compatibility and can culture cells in a feeder-free system, thus avoiding the risks of gene integration and gene contamination. The transcription factor components used are simple, and no microRNA needs to be added during the reprogramming process. There is no need to use expensive transfection reagents, which reduces costs. The present invention further provides nanoliposomes of reprogramming factors prepared using cationic lipid SM102. Compared with the method using conventional transfection reagents, the reprogramming efficiency is significantly improved, and a simpler reprogramming gene formulation is used.

The present invention provides a simple and effective reprogramming method for the fields of regenerative medicine and cells and brings new breakthroughs in disease treatment and tissue regeneration. Further research and optimization will lay a solid foundation for clinical application and bring better quality of life and hope to patients.

Although the present invention has been described in detail with reference to examples thereof, these examples are provided for illustration rather than limitation of the present invention. Other embodiments that can be made in accordance with the principles of the present invention all fall within the scope defined by the claims of the present invention.

Sequences involved in the present invention:
Human Oct3/4 amino acid sequence (SEQ ID NO: 1) 
MAGHLASDFAFSPPPGGGGDGPGGPEPGWVDPRTWLSFQGPPGGPGIGPGVGPGSEVWGI
PPCPPPYEFCGGMAYCGPQVGVGLVPQGGLETSQPEGEAGVGVESNSDGASPEPCTVTPG
AVKLEKEKLEQNPEESQDIKALQKELEQFAKLLKQKRITLGYTQADVGLTLGVLFGKVFSQ
TTICRFEALQLSFKNMCKLRPLLQKWVEEADNNENLQEICKAETLVQARKRKRTSIENRVR
GNLENLFLQCPKPTLQQISHIAQQLGLEKDVVRVWFCNRRQKGKRSSSDYAQREDFEAAG
SPFSGGPVSFPLAPGPHFGTPGYGSPHFTALYSSVPFPEGEAFPPVSVTTLGSPMHSN
Human Klf4 amino acid sequence (SEQ ID NO: 2) 
MAVSDALLPSFSTFASGPAGREKTLRQAGAPNNRWREELSHMKRLPPVLPGRPYDLAAAT
VATDLESGGAGAACGGSNLAPLPRRETEEFNDLLDLDFILSNSLTHPPESVAATVSSSASASS
SSSPSSSGPASAPSTCSFTYPIRAGNDPGVAPGGTGGGLLYGRESAPPPTAPFNLADINDVSP
SGGFVAELLRPELDPVYIPPQQPQPPGGGLMGKFVLKASLSAPGSEYGSPSVISVSKGSPDG
 
SHPVVVAPYNGGPPRTCPKIKQEAVSSCTHLGAGPPLSNGHRPAAHDFPLGRQLPSRTTPTL
GLEEVLSSRDCHPALPLPPGFHPHPGPNYPSFLPDQMQPQVPPLHYQELMPPGSCMPEEPK
PKRGRRSWPRKRTATHTCDYAGCGKTYTKSSHLKAHLRTHTGEKPYHCDWDGCGWKFA
RSDELTRHYRKHTGHRPFQCQKCDRAFSRSDHLALHMKRHF
Human Sox2 amino acid sequence (SEQ ID NO: 3)
Human cMyc amino acid sequence (SEQ ID NO: 4) 
MGPLNVSFTNRNYDLDYDSVQPYFYCDEEENFYQQQQQSELQPPAPSEDIWKKFELLPTPP
LSPSRRSGLCSPSYVAVTPFSLRGDNDGGGGSFSTADQLEMVTELLGGDMVNQSFICDPDD
ETFIKNIIIQDCMWSGFSAAAKLVSEKLASYQAARKDSGSPNPARGHSVCSTSSLYLQDLSA
AASECIDPSVVFPYPLNDSSSPKSCASQDSSAFSPSSDSLLSSTESSPQGSPEPLVLHEETPPT
TSSDSEEEQEDEEEIDVVSVEKRQAPGKRSESGSPSAGGHSKPPHSPLVLKRCHVSTHQHN
YAAPPSTRKDYPAAKRVKLDSVRVLRQISNNRKCTSPRSSDTEENVKRRTHNVLERQRRNE
LKRSFFALRDQIPELENNEKAPKVVILKKATAYILSVQAEEQKLISEEDLLRKRREQLKHKL
EQLRNSCA
Human Lin28 amino acid sequence (SEQ ID NO: 5) 
MGSVSNQQFAGGCAKAAEEAPEEAPEDAARAADEPQLLHGAGICKWFNVRMGFGFLSM
TARAGVALDPPVDVFVHQSKLHMEGFRSLKEGEAVEFTFKKSAKGLESIRVTGPGGVFCIG
SERRPKGKSMQKRRSKGDRCYNCGGLDHHAKECKLPPQPKKCHFCQSISHMVASCPLKA
QQGPSAQGKPTYFREEEEEIHSPTLLPEAQN
Human Glis amino acid sequence (SEQ ID NO: 6) 
MAEARTSLSAHCRGPLATGLHPDLDLPGRSLATPAPSCYLLGSEPSSGLGLQPETHLPEGSL
KRCCVLGLPPTSPASSSPCASSDVTSIIRSSQTSLVTCVNGLRSPPLTGDLGGPSKRARPGPAS
TDSHEGSLQLEACRKASFLKQEPADEFSELFGPHQQGLPPPYPLSQLPPGPSLGGLGLGLAG
RVVAGRQACRWVDCCAAYEQQEELVRHIEKSHIDQRKGEDFTCFWAGCVRRYKPFNARY
KLLIHMRVHSGEKPNKCMFEGCSKAFSRLENLKIHLRSHTGEKPYLCQHPGCQKAFSNSSD
 
RAKHQRTHLDTKPYACQIPGCSKRYTDPSSLRKHVKAHSAKEQQVRKKLHAGPDTEADV
LTECLVLQQLHTSTQLAASDGKGGCGLGQELLPGVYPGSITPHNGLASGLLPPAHDVPSRH
HPLDATTSSHHHLSPLPMAESTRDGLGPGLLSPIVSPLKGLGPPPLPPSSQSHSPGGQPFPTL
PSKPSYPPFQSPPPPPLPSPQGYQGSFHSIQSCFPYGDCYRMAEPAAGGDGLVGETHGFNPL
RPNGYHSLSTPLPATGYEALAEASCPTALPQQPSEDVVSSGPEDCGFFPNGAFDHCLGHIPSI
YTDT
Human Oct3/4 mRNA sequence (SEQ ID NO: 7) 
ggaaaaaggaaagugcacuuggaagagauccaagugggcaacuugaagaacaagugccaaauagcacuucugucaugcuggau
gucagggcucuuuguccacuuuguauagccgcuggcuuauagaaggugcucgauaaaucucuugaauuuaaaaaucaauuagg
augccucuauagugaaaaagauacaguaaagaugagggauaaucaauuaaaaaugaguaaguacacacaaagcacuuuaucca
uucuuaugacaccuguuacuuuuuugcuguguuuguguguaugcaugccauguuauaguuugugggacccucaaagcaagcu
ggggagaguauauacugaauuuagcuucugagacaugaugcucuuccuuuuuaauuaacccagaacuuagcagcuuaucuauu
ucucuaaucucaaaacauccuuaaacugggggugauacuugagugagagaauuuugcagguauuaaaugaacuaucuucuuuu
uuuuuuuucuuugagacagagucuugcucugucacccaggcuggagugcaguggcgugaucucagcucacugcaaccuccgcc
ucccggguucaagugauucuccugccucagccuccugaguagcugggauuacagucccaggacaucaaagcucugcagaaagaa
cucgagcaauuugccaagcuccugaagcagaagaggaucacccugggauauacacaggccgauguggggcucacccuggggguu
cuauuugggaagguauucagccaaacgaccaucugccgcuuugaggcucugcagcuuagcuucaagaacauguguaagcugcgg
cccuugcugcagaaguggguggaggaagcugacaacaaugaaaaucuucaggagauaugcaaagcagaaacccucgugcaggcc
cgaaagagaaagcgaaccaguaucgagaaccgagugagaggcaaccuggagaauuuguuccugcagugcccgaaacccacacugc
agcagaucagccacaucgcccagcagcuugggcucgagaaggaugugguccgagugugguucuguaaccggcgccagaagggca
agcgaucaagcagcgacuaugcacaacgagaggauuuugaggcugcugggucuccuuucucagggggaccaguguccuuuccu
cuggccccagggccccauuuugguaccccaggcuaugggagcccucacuucacugcacuguacuccucggucccuuucccugag
ggggaagccuuucccccugucuccgucaccacucugggcucucccaugcauucaaacugaggugccugcccuucuaggaauggg
ggacagggggaggggaggagcuagggaaagaaaaccuggaguuugugccaggguuuuugggauuaaguucuucauucacuaag
gaaggaauugggaacacaaagggugggggcaggggaguuuggggcaacugguuggagggaaggugaaguucaaugaugcucu
ugauuuuaaucccacaucauguaucacuuuuuucuuaaauaaagaagccugggacacaguagauagacacacuua
Human Klf4 mRNA sequence (SEQ ID NO: 8) 
ggcaguuucccgaccagagagaacgaacgugucugcgggcgcgcggggagcagaggcgguggcgggcggcggcaccggga
gccgccgagugacccucccccgccccucuggccccccacccucccacccgcccguggcccgcgcccauggccgcgcgcgcuccaca
caacucaccggaguccgcgccuugcgccgccgaccaguucgcagcuccgcgccacggcagccagucucaccuggcggcaccgccc
gcccaccgccccggccacagccccugcgcccacggcagcacucgaggcgaccgcgacaguggugggggacgcugcugaguggaa
gagagcgcagcccggccaccggaccuacuuacucgccuugcugauugucuauuuuugcguuuacaacuuuucuaagaacuuuu
guauacaaaggaacuuuuuaaaaaagacgcuuccaaguuauauuuaauccaaagaagaaggaucucggccaauuugggguuuug
 
gguuuuggcuucguuucuucucuucguugacuuugggguucaggugccccagcugcuucgggcugccgaggaccuucugggc
ccccacauuaaugaggcagccaccuggcgagucugacauggcugucagcgacgcgcugcucccaucuuucuccacguucgcguc
uggcccggcgggaagggagaagacacugcgucaagcaggugccccgaauaaccgcuggcgggaggagcucucccacaugaagcg
acuucccccagugcuucccggccgccccuaugaccuggcggcggcgaccguggccacagaccuggagagcggcggagccggugc
ggcuugcggcgguagcaaccuggcgccccuaccucggagagagaccgaggaguucaacgaucuccuggaccuggacuuuauucu
cuccaauucgcugacccauccuccggagucaguggccgccaccguguccucgucagcgucagccuccucuucgucgucgccguc
gagcagcggcccugccagcgcgcccuccaccugcagcuucaccuauccgauccgggccgggaacgacccgggcguggcgccggg
cggcacgggcggaggccuccucuauggcagggaguccgcucccccuccgacggcucccuucaaccuggcggacaucaacgacgu
gagccccucgggcggcuucguggccgagcuccugcggccagaauuggacccgguguacauuccgccgcagcagccgcagccgcc
agguggcgggcugaugggcaaguucgugcugaaggcgucgcugagcgccccuggcagcgaguacggcagcccgucggucauca
gcgucagcaaaggcagcccugacggcagccacccgguggugguggcgcccuacaacggcgggccgccgcgcacgugccccaaga
ucaagcaggaggcggucucuucgugcacccacuugggcgcuggacccccucucagcaauggccaccggccggcugcacacgacu
ucccccuggggcggcagcuccccagcaggacuaccccgacccugggucuugaggaagugcugagcagcagggacugucacccug
cccugccgcuuccucccggcuuccauccccacccggggcccaauuacccauccuuccugcccgaucagaugcagccgcaaguccc
gccgcuccauuaccaagagcucaugccacccgguuccugcaugccagaggagcccaagccaaagaggggaagacgaucguggccc
cggaaaaggaccgccacccacacuugugauuacgcgggcugcggcaaaaccuacacaaagaguucccaucucaaggcacaccugc
gaacccacacaggugagaaaccuuaccacugugacugggacggcuguggauggaaauucgcccgcucagaugaacugaccaggc
acuaccguaaacacacggggcaccgcccguuccagugccaaaaaugcgaccgagcauuuuccaggucggaccaccucgccuuaca
caugaagaggcauuuuuaaaucccagacaguggauaugacccacacugccagaagagaauucaguauuuuuuacuuuucacacu
gucuucccgaugagggaaggagcccagccagaaagcacuacaaucauggucaaguucccaacugagucaucuugugaguggaua
aucaggaaaaaugaggaauccaaaagacaaaaaucaaagaacagauggggucugugacuggaucuucuaucauuccaauucuaaa
uccgacuugaauauuccuggacuuacaaaaugccaagggggugacuggaaguuguggauaucaggguauaaauuauauccgug
aguugggggagggaagaccagaauucccuugaauuguguauugaugcaauauaagcauaaaagaucaccuuguauucucuuua
ccuucuaaaagccauuauuaugauguuagaagaagaggaagaaauucagguacagaaaacauguuuaaauagccuaaaugaugg
ugcuuggugagucuugguucuaaagguaccaaacaaggaagccaaaguuuucaaacugcugcauacuuugacaaggaaaaucua
uauuugucuuccgaucaacauuuaugaccuaagucagguaauauaccugguuuacuucuuuagcauuuuuaugcagacagucu
guuaugcacugugguuucagaugugcaauaauuuguacaaugguuuauucccaaguaugccuuaagcagaacaaauguguuuu
ucuauauaguuccuugccuuaauaaauauguaauauaaauuuaagcaaacgucuauuuuguauauuuguaaacuacaaaguaaa
augaacauuuuguggaguuuguauuuugcauacucaaggugagaauuaaguuuuaaauaaaccuauaauauuuuaucugaa
Human Sox2 mRNA sequence (SEQ ID NO: 9) 
gaugguugucuauuaacuuguucaaaaaaguaucaggaguugucaaggcagagaagagaguguuugcaaaggggaaaguag
uuugcugccucuuuaagacuaggacugagagaaagaagaggagagagaaagaaagggagagaaguuugagccccaggcuuaagc
cuuuccaaaaaauaauaauaacaaucaucggcggcggcaggaucggccagaggaggagggaagcgcuuuuuuugauccugauuc
caguuugccucucucuuuuuuucccccaaauuauucuucgccugauuuuccucgcggagcccugcgcucccgacacccccgccc
 
gccuccccuccuccucuccccccgcccgcgggccccccaaagucccggccgggccgagggucggcggccgccggcgggccgggcc
cgcgcacagcgcccgcauguacaacaugauggagacggagcugaagccgccgggcccgcagcaaacuucggggggcggcggcgg
caacuccaccgcggcggcggccggcggcaaccagaaaaacagcccggaccgcgucaagcggcccaugaaugccuucauggugugg
ucccgcgggcagcggcgcaagauggcccaggagaaccccaagaugcacaacucggagaucagcaagcgccugggcgccgagugg
aaacuuuugucggagacggagaagcggccguucaucgacgaggcuaagcggcugcgagcgcugcacaugaaggagcacccggau
uauaaauaccggccccggcggaaaaccaagacgcucaugaagaaggauaaguacacgcugcccggcgggcugcuggcccccggcg
gcaauagcauggcgagcggggucggggugggcgccggccugggcgcgggcgugaaccagcgcauggacaguuacgcgcacaug
aacggcuggagcaacggcagcuacagcaugaugcaggaccagcugggcuacccgcagcacccgggccucaaugcgcacggcgcag
cgcagaugcagcccaugcaccgcuacgacgugagcgcccugcaguacaacuccaugaccagcucgcagaccuacaugaacggcuc
gcccaccuacagcauguccuacucgcagcagggcaccccuggcauggcucuuggcuccauggguucgguggucaaguccgaggc
cagcuccagccccccugugguuaccucuuccucccacuccagggcgcccugccaggccggggaccuccgggacaugaucagcau
guaucuccccggcgccgaggugccggaacccgccgcccccagcagacuucacaugucccagcacuaccagagcggcccggugccc
ggcacggccauuaacggcacacugccccucucacacaugugagggccggacagcgaacuggaggggggagaaauuuucaaagaa
aaacgagggaaaugggaggggugcaaaagaggagaguaagaaacagcauggagaaaacccgguacgcucaaaaagaaaaaggaaa
aaaaaaaaucccaucacccacagcaaaugacagcugcaaaagagaacaccaaucccauccacacucacgcaaaaaccgcgaugccga
caagaaaacuuuuaugagagagauccuggacuucuuuuugggggacuauuuuuguacagagaaaaccuggggaggguggggag
ggcgggggaauggaccuuguauagaucuggaggaaagaaagcuacgaaaaacuuuuuaaaaguucuagugguacgguaggagc
uuugcaggaaguuugcaaaagucuuuaccaauaauauuuagagcuagucuccaagcgacgaaaaaaauguuuuaauauuugcaa
gcaacuuuuguacaguauuuaucgagauaaacauggcaaucaaaauguccauuguuuauaagcugagaauuugccaauauuuuu
caaggagaggcuucuugcugaauuuugauucugcagcugaaauuuaggacaguugcaaacgugaaaagaagaaaauuauucaaa
uuuggacauuuuaauuguuuaaaaauuguacaaaaggaaaaaauuagaauaaguacuggcgaaccaucucuguggucuuguuu
aaaaagggcaaaaguuuuagacuguacuaaauuuuauaacuuacuguuaaaagcaaaaauggccaugcagguugacaccguugg
uaauuuauaauagcuuuuguucgaucccaacuuuccauuuuguucagauaaaaaaaaccaugaaauuacuguguuugaaauauu
uucuuaugguuuguaauauuucuguaaauuuauugugauauuuuaagguuuuccccccuuuauuuuccguaguuguauuuua
aaagauucggcucuguauuauuugaaucagucugccgagaauccauguauauauuugaacuaauaucauccuuauaacagguac
auuuucaacuuaaguuuuuacuccauuaugcacaguuugagauaaauaaauuuuugaaauauggacacugaaa
Human cMyc mRNA sequence (SEQ ID NO: 10) 
ggaguuuauucauaacgcgcucuccaaguauacguggcaaugcguugcuggguuauuuuaaucauucuaggcaucguuuuccu

ccuuaugccucuaucauuccucccuaucuacacuaacaucccacgcucugaacgcgcgcccauuaauacccuucuuuccuccacu

cucccugggacucuugaucaaagcgcggcccuuuccccagccuuagcgaggcgcccugcagccugguacgcgcguggcguggcg

gugggcgcgcagugcguucucgguguggagggcagcuguuccgccugcgaugauuuauacucacaggacaaggaugcgguuug

ucaaacaguacugcuacggaggagcagcagagaaagggagaggguuugagagggagcaaaagaaaaugguaggcgcgcguaguu

aauucaugcggcucucuuacucuguuuacauccuagagcuagagugcucggcugcccggcugagucuccuccccaccuucccca

cccuccccacccuccccauaagcgccccucccggguucccaaagcagagggcgugggggaaaagaaaaaagauccucucucgcua
 
aucuccgcccaccggcccuuuauaaugcgagggucuggacggcugaggacccccgagcugugcugcucgcggccgccaccgccg
ggccccggccgucccuggcuccccuccugccucgagaagggcagggcuucucagaggcuuggcgggaaaaagaacggagggagg
gaucgcgcugaguauaaaagccgguuuucggggcuuuaucuaacucgcuguaguaauuccagcgagaggcagagggagcgagc
gggcggccggcuaggguggaagagccgggcgagcagagcugcgcugcgggcguccugggaagggagauccggagcgaauaggg
ggcuucgccucuggcccagcccucccgcugaucccccagccagcgguccgcaacccuugccgcauccacgaaacuuugcccauag
cagcgggcgggcacuuugcacuggaacuuacaacacccgagcaaggacgcgacucucccgacgcggggaggcuauucugcccau
uuggggacacuuccccgccgcugccaggacccgcuucucugaaaggcucuccuugcagcugcuuagacgcuggauuuuuuucg
gguaguggaaaaccagccucccgcgacgaugccccucaacguuagcuucaccaacaggaacuaugaccucgacuacgacucggug
cagccguauuucuacugcgacgaggaggagaacuucuaccagcagcagcagcagagcgagcugcagcccccggcgcccagcgag
gauaucuggaagaaauucgagcugcugcccaccccgccccuguccccuagccgccgcuccgggcucugcucgcccuccuacguu
gcggucacacccuucucccuucggggagacaacgacggcgguggcgggagcuucuccacggccgaccagcuggagauggugacc
gagcugcugggaggagacauggugaaccagaguuucaucugcgacccggacgacgagaccuucaucaaaaacaucaucauccag
gacuguauguggagcggcuucucggccgccgccaagcucgucucagagaagcuggccuccuaccaggcugcgcgcaaagacagc
ggcagcccgaaccccgcccgcggccacagcgucugcuccaccuccagcuuguaccugcaggaucugagcgccgccgccucagagu
gcaucgaccccucgguggucuuccccuacccucucaacgacagcagcucgcccaaguccugcgccucgcaagacuccagcgccuu
cucuccguccucggauucucugcucuccucgacggaguccuccccgcagggcagccccgagccccuggugcuccaugaggagac
accgcccaccaccagcagcgacucugaggaggaacaagaagaugaggaagaaaucgauguuguuucuguggaaaagaggcaggc
uccuggcaaaaggucagagucuggaucaccuucugcuggaggccacagcaaaccuccucacagcccacugguccucaagaggug
ccacgucuccacacaucagcacaacuacgcagcgccucccuccacucggaaggacuauccugcugccaagagggucaaguuggac
agugucagaguccugagacagaucagcaacaaccgaaaaugcaccagccccagguccucggacaccgaggagaaugucaagaggc
gaacacacaacgucuuggagcgccagaggaggaacgagcuaaaacggagcuuuuuugcccugcgugaccagaucccggaguugg
aaaacaaugaaaaggcccccaagguaguuauccuuaaaaaagccacagcauacauccuguccguccaagcagaggagcaaaagcu
cauuucugaagaggacuuguugcggaaacgacgagaacaguugaaacacaaacuugaacagcuacggaacucuugugcguaagg
aaaaguaaggaaaacgauuccuucuaacagaaauguccugagcaaucaccuaugaacuuguuucaaaugcaugaucaaaugcaac
cucacaaccuuggcugagucuugagacugaaagauuuagccauaauguaaacugccucaaauuggacuuugggcauaaaagaac
uuuuuuaugcuuaccaucuuuuuuuuuucuuuaacagauuuguauuuaagaauuguuuuuaaaaaauuuuaagauuuacacaa
uguuucucuguaaauauugccauuaaauguaaauaacuuuaauaaaacguuuauagcaguuacacagaauuucaauccuaguau
auaguaccuaguauuauagguacuauaaacccuaauuuuuuuuauuuaaguacauuuugcuuuuuaaaguugauuuuuuucua
uuguuuuuagaaaaaauaaaauaacuggcaaauauaucauugagccaaaucuuaaguugugaauguuuuguuucguuucuucc
cccucccaaccaccaccaucccuguuuguuuucaucaauugccccuucagaggguggucuuaagaaaggcaagaguuuuccucu
guugaaaugggucugggggccuuaaggucuuuaaguucuuggagguucuaagaugcuuccuggagacuaugauaacagccaga
guugacaguuagaaggaauggcagaaggcaggugagaaggugagagguaggcaaaggagauacaagaggucaaagguagcagu
uaaguacacaaagaggcauaaggacuggggaguugggaggaaggugaggaagaaacuccuguuacuuuaguuaaccagugccag
uccccugcucacuccaaacccaggaauucugcccaguugauggggacacggugggaaccagcuucugcugccuucacaaccagg
cgccaguccuguccauggguuaucucgcaaaccccagaggaucucugggaggaaugcuacuauuaacccuauuucacaaacaag
 
gaaauagaagagcucaaagagguuauguaacuuaucuguagccacgcagauaauacaaagcagcaaucuggacccauucuguuc
aaaacacuuaacccuucgcuaucaugccuugguucaucugggucuaaugugcugagaucaagaagguuuaggaccuaauggaca
gacucaagucauaacaaugcuaagcucuauuugugucccaagcacuccuaagcauuuuaucccuaacucuacaucaaccccauga
aggagauacuguugauuuccccauauuagaaguagagagggaagcugaggcacacaaagacucauccacaugcccaagauucac
ugauagggaaaaguggaagcgagauuugaacccaggcuguuuacuccuaaccuguccaagccaccucucagacgacgguaggaa
ucagcuggcugcuugugaguacaggaguuacaguccaguggguuauguuuuuuaagucucaacaucuaagccuggucaggcau
caguuccccuuuuuuugugauuuauuuuguuuuuauuuuguuguucauuguuuaauuuuuccuuuuacaaugagaaggucac
caucuugacuccuaccuuagccauuuguugaaucagacucaugacggcuccugggaagaagccaguucagaucauaaaauaaaa
cauauuuauucuuugucaugggagucauuauuuuagaaacuacaaacucuccuugcuuccauccuuuuuuacauacucaugaca
caugcucauccugaguccuugaaaagguauuuuugaacauguguauuaauuauaagccucugaaaaccuauggcccaaaccaga
aaugauguugauuauauagguaaaugaaggaugcuauugcuguucuaauuaccucauugucucagucucaaaguaggucuuca
gcucccuguacuuugggauuuuaaucuaccaccacccauaaaucaauaaauaauuacuuucuuuga
Human Lin28 mRNA sequence (SEQ ID NO: 11) 
ccuuugccuucggacuucuccggggccagcagccgcccgaccaggggcccggggccacgggcucagccgacgaccaugggcucc
guguccaaccagcaguuugcagguggcugcgccaaggcggcagaagaggcgcccgaggaggcgccggaggacgcggcccgggcg
gcggacgagccucagcugcugcacggugcgggcaucuguaagugguucaacgugcgcaugggguucggcuuccuguccaugac
cgcccgcgccggggucgcgcucgaccccccaguggaugucuuugugcaccagaguaagcugcacauggaaggguuccggagcuu
gaaggagggugaggcaguggaguucaccuuuaagaagucagccaagggucuggaauccauccgugucaccggaccugguggag
uauucuguauugggagugagaggcggccaaaaggaaagagcaugcagaagcgcagaucaaaaggagacaggugcuacaacugug
gaggucuagaucaucaugccaaggaaugcaagcugccaccccagcccaagaagugccacuucugccagagcaucagccauauggu
agccucauguccgcugaaggcccagcagggcccuagugcacagggaaagccaaccuacuuucgagaggaagaagaagaaauccac
agcccuacccugcucccggaggcacagaauugagccacaaugggugggggcuauucuuuugcuaucaggaaguuuugaggagc
aggcagaguggagaaagugggaauagggugcauuggggcuaguuggcacugccauguaucucaggcuuggguucacaccauca
cccuuucuucccucuagguggggggaaagggugagucaaaggaacuccaaccaugcucuguccaaaugcaagugaggguucug
ggggcaaccaggaggggggaaucacccuacaaccugcauacuuugagucuccauccccagaauuuccagcuuuugaaaguggcc
uggauagggaaguuguuuuccuuuuaaagaaggauauauaauaauucccaugccagagugaaaugauuaaguauaagaccagau
ucauggagccaagccacuacauucuguggaaggagaucucucaggaguaagcauuguuuuuuuuucacaucuuguauccucau
acccacuuuugggauagggugcuggcagcugucccaagcaauggguaaugaugauggcaaaaaggguguuugggggaacagcu
gcagaccugcugcucuaugcucacccccgccccauucugggccaaugugauuuuauuuauuugcucccuuggauacugcaccuu
gggucccacuuucuccaggaugccaacugcacuagcugugugcgaaugacguaucuugugcauuuuaacuuuuuuuccuuaau
auaaauauucugguuuuguauuuuuguauauuuuaaucuaaggcccucauuuccugcacuguguucucagguacaugagcaau
cucagggauagccagcagcagcuccaggucugcgcagcaggaauuacuuuuuguuguuuuugccaccguggagagcaacuauu
uggagugcacagccuauugaacuaccucauuuuugccaauaagagcuggcuuuucugccauaguguccucuugaaacccccucu
gccuugaaaauguuuuaugggagacuagguuuuaacuggguggccccaugacuugauugccuucuacuggaagauugggaauu
 
agucuaaacaggaaauggugguacacagaggcuaggagaggcugggcccggugaaaaggccagagagcaagccaagauuaggug
aggguugucuaauccuauggcacaggacgugcuuuacaucuccagaucuguucuucaccagauuagguuaggccuaccaugug
ccacaggguguguguguguuuguaaaacuagaguugcuaaggauaaguuuaaagaccaauaccccuguacuuaauccugugcu
gucgagggauggauauaugaaguaaggugagauccuuaaccuuucaaaauuuucggguuccagggagacacacaagcgagggu
uuuguggugccuggagccuguguccugcccugcuacaguagugauuaauagugucaugguagcuaaaggagaaaaaggggguu
ucguuuacacgcugugagaucaccgcaaaccuaccuuacuguguugaaacgggacaaaugcaauagaacgcauuggguggugug
ugucugauccuggguucuugucuccccuaaaugcugccccccaaguuacuguauuugucugggcuuuguaggacuucacuacg
uugauugcuagguggccuaguuuguguaaauauaauguauuggucuuucuccguguucuuuggggguuuuguuuacaaacuu
cuuuuuguauugagagaaaaauagccaaagcaucuuugacagaagguucugcaccaggcaaaaagaucugaaacauuaguuugg
ggggcccucuucuuaaaguggggaucuugaaccauccuuucuuuuguauuccccuuccccuauuaccuauuagaccagaucuuc
uguccuaaaaacuugucuucuacccugcccucuuuucuguucacccccaaaagaaaacuuacacacccacacacauacacauuuc
augcuuggagugucuccacaacucuuaaaugauguaugcaaaaauacugaagcuaggaaaacccuccaucccuuguucccaaccu
ccuaagucaagaccauuaccauuucuuucuuucuuuuuuuuuuuuuuuuaaaauggagucucacugugucacccaggcuggag
ugcaguggcaugaucggcucacugcagccucugccucuuggguucaagugauucuccugccucagccuccugaguagcuggga
uuucaggcacccgccacacucagcuaauuuuuguauuuuuaguagagacgggguuucaccauguuguccaggcuggucuggaa
cuccugaccucaggugaucugcccaccuuggcuucccaaagugcugggauuacaggcaugagccaccaugcugggccaaccauu
ucuugguguauucaugccaaacacuuaagacacugcuguagcccaggcgcgguggcucacaccuguaaucccagcacuuuggaa
ggcugaggcgggcggaucacaaggucacgaguucaaaacuauccuggccaacacagugaaaccccgucucuacuaaaauacaaaa
aaauuagccgggugugguggugcaugccuuuaguccuagcuauucaggaggcugaggcaggggaaucgcuugaacccgagagg
cagagguugcagugagcugagaucgcaccacugcacuccagccugguuacagagcaagacucugucucaaacaaaacaaaacaaa
acaaaaacacacuacuguauuuuggauggaucaaaccuccuuaauuuuaauuucuaauccuaaaguaaagagaugcaauugggg
gccuuccauguagaaaguggggucaggaggccaagaaagggaauaugaauguauauccaagucacucaggaacuuuuaugcagg
ugcuagaaacuuuaugucaaaguggccacaagauuguuuaauaggagacgaacgaauguaacuccauguuuacugcuaaaaacc
aaagcuuuguguaaaaucuugaauuuauggggcgggaggguaggaaagccuguaccugucuguuuuuuuccugauccuuuucc
cucauuccugaacugcaggagacugagccccuuugggcuuuggugaccccaucacugggguguguuuauuugaugguugauuu
ugcuguacuggguacuuccuuucccauuuucuaaucauuuuuuaacacaagcugacucuucccuucccuucuccuuucccugg
gaaaauacaaugaauaaauaaagacuuauugguacgcaaacuguca
Human Glis mRNA sequence (SEQ ID NO: 12) 
acuguguacugagacuggaugcauccuugcaauaaaaaagagguugaucacgacaaaugugaaccccgccguuauaaaaacagcc
aucauggcuguaaaugccaaaaagcagucagucuuguaacuugaaaaaaaaaaaaaaggaauuguagauugugcgcauggacuc
ggagugggggcgguggacaguaagucaugauguuuuggugguaccaccugguugaauuucuucaucugaauaagaagcuccug
ugauguucuggggaggccuuggaaggcuagcgcaucccucauagaaagugaaugggagcuacggacaccguaccccgggcucag
agaagagccugcuggaccuggaccuugcugagggcccuggccccaccugcugccagggccuguuucucccugcaggaagcccac
cgccccgggcucacccccaagcuugugagaggcugcugcauuucccccacccugacaggucaccuagaccccaggccacguaugu
 
gaacggcagccucccaaccacacaacacaucaaacaggaguccuugcccgacuaccaagccauggcagaggcccgcacaucccugu
cugcccacugucggggcccgcuggccacuggccugcacccagaccuggaccucccgggccgaagccucgccaccccugcgccuuc
cugcuaccuucugggcagcgaacccagcucuggccugggccuccagcccgagacccaccuccccgagggcagccugaagcggug
cugcgucuugggccuaccccccaccuccccagccuccuccucacccugugccuccuccgacgucaccuccaucauccgcuccucc
cagacgucucuggucaccuguguaaauggacuccggagccccccucugacgggagaucuggggggcccuuccaagcgggcccgg
ccuggcccugcaucgacggacagccaugagggcagcuugcaacuugaagccugccggaaggcgagcuuccugaagcaggaaccc
gcggaugaguuuucagagcucuuugggccucaccagcagggccugccgccccccuauccccugucucaguugccgccuggccca
agccuuggaggccuggggcugggccuggcaggcaggguggugcccgggcggcaggcgugccgcuggguggacugcugugcag
ccuaugagcagcaggaggagcuggugcggcacaucgagaagagccacaucgaccagcgcaagggcgaggacuucaccugcuucu
gggcuggcugcgugcgccgcuacaagcccuucaacgcccgcuacaagcugcucauccacaugcgagugcacucgggcgagaagc
ccaacaagugcauguuugaaggcugcagcaaggccuucucacggcuggagaaccucaagauccaccugaggagccacacgggcg
agaagccguaccugugccagcacccggguugccagaaggccuucagcaacuccagcgaccgcgccaagcaccagcgcacccaccu
agacacgaagccguacgccugucagaucccuggcugcuccaagcgcuacacagaccccagcucccuccgcaagcacgucaaggcc
cauucagccaaagagcagcaggugcguaagaagcugcaugcgggcccugacaccgaggccgacguccugaccgagugucugguc
cugcagcagcuccacacguccacacagcuggcugccagcgacggcaaggguggcuguggccugggccaggagcugcucccaggu
guguauccuggcuccaucaccccccauaacggacuugcaucgggccuccugcccccagcgcacgacguaccuuccaggcaccacc
cgcuggaugccaccaccaguucccaccaccaucuguccccucugcccauggcugagagcacccgggauggguuggggcccggcc
uccucucaccaauagucagcccccugaaggggcuggggccaccgccgcugcccccauccucucagagccauucuccggggggcca
gcccuuccccacacuccccagcaagccguccuacccacccuuccagagcccuccacccccgccucugcccagcccacaagguuacc
agggcaguuuccacuccauccagaguugcuuccccuauggcgacugcuaccggauggcugaaccagcagccgguggggacggac
uggucggggagacccacgguuucaacccccugcggcccaauggcuaccacagccucagcacgcccuugccugccacaggcuauga
ggcccuggcugaggccucaugccccacagcgcugccacagcagccaucugaagaugugguguccagcggccccgaggacugugg
cuucuuccccaauggagccuuugaccacugccugggccacauccccuccaucuacacagacaccugaaggagcccccacaugcgc
cugcccauccagcacugcagaugccaccucgcccaccugcugucgcucccacccuccgugcaccuagcaggagugccaggccaca
gccggaacagccaggccaugacccaggggagccagcgcugccaccccacccagcgcugccagggagccgccauccgagcuugagc
ugggcgcacagaggugcccgccaggaucuguggcccuguaacauucccucgaucuugucuucccguuccuccccgcagugguu
uugaaaucacagaccucguguauauaaaauaugcagaacuuguuuuccguuccccugccaguuuuauauuuuugguuuuacaa
gaaaaaacauuaaaaacuggaaagga
Human Nanog amino acid sequence (SEQ ID NO: 13) 
MSVDPACPQSLPCFEASDCKESSPMPVICGPEENYPSLQMSSAEMPHTETVSPLPSSMDLLI
QDSPDSSTSPKGKQPTSAEKSVAKKEDKVPVKKQKTRTVFSSTQLCVLNDRFQRQKYLSL
QQMQELSNILNLSYKQVKTWFQNQRMKSKRWQKNNWPKNSNGVTQKASAPTYPSLYSS
YHQGCLVNPTGNLPMWSNQTWNNSTWSNQTQNIQSWSNHSWNTQTWCTQSWNNQAW
NSPFYNCGEESLQSCMQFQPNSPASDLEAALEAAGEGLNVIQQTTRYFSTPQTMDLFLNYS
 MNMQPEDV
Human Nanog mRNA sequence (SEQ ID NO: 14)

auaaaucuagagacuccaggauuuuaacguucugcuggacugagcugguugccucauguuauugcaggcaacucacuuuau
cccaauuucuugauacuuuuccuucuggagguccuauuucucuaacaucuuccagaaaagucuuaaagcugccuuaaccuuuuu
uccaguccaccucuuaaauuuuuuccuccucuuccucuauacuaacaugaguguggauccagcuuguccccaaagcuugccuug
cuuugaagcauccgacuguaaagaaucuucaccuaugccugugauuugugggccugaagaaaacuauccauccuugcaaauguc
uucugcugagaugccucacacggagacugucucuccucuuccuuccuccauggaucugcuuauucaggacagcccugauucuuc
caccagucccaaaggcaaacaacccacuucugcagagaagagugucgcaaaaaaggaagacaaggucccggucaagaaacagaaga
ccagaacuguguucucuuccacccagcuguguguacucaaugauagauuucagagacagaaauaccucagccuccagcagaugc
aagaacucuccaacauccugaaccucagcuacaaacaggugaagaccugguuccagaaccagagaaugaaaucuaagagguggca
gaaaaacaacuggccgaagaauagcaauggugugacgcagaaggccucagcaccuaccuaccccagccuuuacucuuccuaccac
cagggaugccuggugaacccgacugggaaccuuccaauguggagcaaccagaccuggaacaauucaaccuggagcaaccagaccc
agaacauccaguccuggagcaaccacuccuggaacacucagaccuggugcacccaauccuggaacaaucaggccuggaacagucc
cuucuauaacuguggagaggaaucucugcaguccugcaugcaguuccagccaaauucuccugccagugacuuggaggcugccuu
ggaagcugcuggggaaggccuuaauguaauacagcagaccacuagguauuuuaguacuccacaaaccauggauuuauuccuaaa
cuacuccaugaacaugcaaccugaagacgugugaagaugagugaaacugauauuacucaauuucagucuggacacuggcugaau
ccuuccucuccccuccucccaucccucauaggauuuuucuuguuuggaaaccacguguucugguuuccaugaugcccauccagu
caaucucauggaggguggaguaugguuggagccuaaucagcgagguuucuuuuuuuuuuuuuuuccuauuggaucuuccugg
agaaaauacuuuuuuuuuuuuuuuuuuugaaacggagucuugcucugucgcccaggcuggagugcaguggcgcggucuuggc
ucacugcaagcuccgucucccggguucacgccauucuccugccucagccucccgagcagcugggacuacaggcgcccgccaccuc
gcccggcuaauauuuuguauuuuuaguagagacgggguuucacuguguuagccaggauggucucgaucuccugaccuugugau
ccacccgccucggccucccuaacagcugggauuuacaggcgugagccaccgcgcccugccuagaaaagacauuuuaauaaccuug
gcugccgucucuggcuauagauaaguagaucuaauacuaguuuggauaucuuuaggguuuagaaucuaaccucaagaauaagaa
auacaaguacaaauuggugaugaagauguauucguauuguuugggauugggaggcuuugcuuauuuuuuaaaaacuauugag
guaaaggguuaagcuguaacauacuuaauugauuucuuaccguuuuuggcucuguuuugcuauauccccuaauuuguugguu
gugcuaaucuuuguagaaagaggucucguauuugcugcaucguaaugacaugaguacugcuuuaguugguuuaaguucaaaug
aaugaaacaacuauuuuuccuuuaguugauuuuac

## Claims

1. A reprogramming method for an induced pluripotent stem cell, comprises:
S1: preparing a nanoliposome mRNA-LNP by using a cationic lipid to encapsulate a mRNA cocktail of reprogramming factors; and
S2: inducing reprogramming of a somatic cell using the nanoliposome mRNA-LNP to obtain a reprogrammed induced pluripotent stem cell.

2. The reprogramming method of claim 1, wherein, the encapsulation with the nanoliposome mRNA-LNP further comprises other lipids or non-lipids in addition to the cationic lipid.

3. The method of claim 1, wherein, the cationic lipid is SM102.

4. The method of claim 1, wherein, the reprogrammed induced pluripotent stem cell is configured to be cultured in a feeder-free system.

5. The method of claim 4, wherein, a matrigel of the feeder-free system is one or more selected from a basement membrane matrix with an animal-derived component, a vitronectin adhesion matrix without an animal-derived component, and a recombinant human laminin matrix without an animal-derived component.

6. The method of claim 1, wherein, the mRNA cocktail of the reprogramming factors comprises:
1) mRNA-hOCT3/4, mRNA-hSox2, mRNA-hKlf4, and mRNA-hcMyc; and
2) one or more selected from mRNA-hLin28, mRNA-hNanog, and mRNA-hGlis.

7. The method of claim 6, wherein, the mRNA cocktail of the reprogramming factors is a cocktail of mRNA-hOCT3/4, mRNA-hSox2, mRNA-hKlf4, mRNA-hcMyc, and mRNA-hGlis.

8. The method of claim 7, wherein, the mRNA-hOCT3/4 comprises a sequence as set forth in SEQ ID NO: 7, the mRNA-hSox2 comprises a sequence as set forth in SEQ ID NO: 9, the mRNA-hKlf4 comprises a sequence as set forth in SEQ ID NO: 8, the mRNA-hcMyc comprises a sequence as set forth in SEQ ID NO: 10, and the mRNA-hGlis comprises a sequence as set forth in SEQ ID NO: 12.

9. The method of claim 6, wherein, the mRNA cocktail of the reprogramming factors is a cocktail of mRNA-hOCT3/4, mRNA-hSox2, mRNA-hKlf4, mRNA-hcMyc, and mRNA-hLin28.

10. The method of claim 9, wherein, the mRNA-hOCT3/4 comprises a sequence as set forth in SEQ ID NO: 7, the mRNA-hSox2 comprises a sequence as set forth in SEQ ID NO: 9, the mRNA-hKlf4 comprises a sequence as set forth in SEQ ID NO: 8, the mRNA-hcMyc comprises a sequence as set forth in SEQ ID NO: 10, and the mRNA-hLin28 comprises a sequence as set forth in SEQ ID NO: 11.

11. The method of claim 1, wherein, the somatic cell is one or more selected from a skin fibroblast, an oral epithelial cell, a hepatocyte, a gastric cell, a keratinocyte, an adipocyte, a muscle cell, or a blood cell.

12. The method of claim 11, wherein, the somatic cell is derived from a mammal.

13. The method of claim 12, wherein, the somatic cell is derived from a monkey, a bovine, an equine, a sheep, or a murine.

14. The method of claim 12, wherein, the somatic cell is derived from a human.

15. The method of claim 14, wherein, the somatic cell is a human dermal fibroblast.
